(19) **Europäisches Patentamt / European Patent Office / Office européen des brevets**

(11) **EP 3 552 703 A1**

(12) **EUROPÄISCHE PATENTANMELDUNG**

(43) Veröffentlichungstag:
**16.10.2019 Patentblatt 2019/42**

(51) Int Cl.:
**B01L 1/00** *(2006.01)* **C12M 1/00** *(2006.01)*

(21) Anmeldenummer: **18166337.8**

(22) Anmeldetag: **09.04.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Eppendorf AG**
**22339 Hamburg (DE)**

(72) Erfinder:
• **Fitzer, Jan**
**22339 Hamburg (DE)**

• **Abel, Philipp**
**22339 Hamburg (DE)**
• **Mensch, Sören**
**22339 Hamburg (DE)**
• **Timmann, Lutz**
**22339 Hamburg (DE)**

(74) Vertreter: **Kirchner, Christian**
**Wallinger Ricker Schlotter Tostmann**
**Patent- und Rechtsanwälte Partnerschaft mbB**
**Zweibrückenstrasse 5 - 7**
**80331 München (DE)**

(54) **LABORTEMPERIERVORRICHTUNGEN**

(57) Die Erfindung betrifft Labortemperiervorrichtungen zum Lagern von Laborproben. Sie betrifft insbesondere Inkubatoren für das Wachstum von Zellkulturen. Es werden effiziente Maßnahmen zur thermischen Entkopplung von Kammer (3) und Gehäuse (2) der Labortemperiervorrichtung beschrieben.

Fig. 5e

EP 3 552 703 A1

**Beschreibung**

Die Erfindung betrifft eine Labortemperiervorrichtung zum Lagern von Laborproben bei einer Zieltemperatur. Sie betrifft insbesondere einen Inkubator für das Wachstum von Zellkulturen.

Labortemperiervorrichtungen werden benötigt, um Laborproben in einer abgeschirmten Umgebung bei einer bestimmten Zieltemperatur aufzubewahren. Mit Inkubatoren werden in biologischen und medizinischen Laboratorien Zellen in Zellkultur unter kontrollierten Umgebungsbedingungen gehalten, um so das Wachstum lebender Zellen *in vitro* zu ermöglichen. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen müssen in $CO_2$-Inkubatoren kultiviert werden. Die Atmosphäre wird durch Luft mit einem bestimmten $CO_2$- und $O_2$-Gehalt und einer bestimmten Luftfeuchtigkeit gebildet, eine geeignete Temperatur ist oftmals 37 °C, wobei diese Parameter meist einstellbar sind. Um die für Zellen jeweils benötigten Umgebungsbedingungen zuverlässig zu gewährleisten, ist eine homogene Temperaturverteilung bzw. ein homogenes Klima in der Inkubatorkammer sowie eine Störungsunempfindlichkeit gegenüber äußeren Einflüssen wünschenswert.

Solche Labortemperiervorrichtungen weisen eine Kammer zur Aufnahme der zu temperierenden Laborproben auf, diese Kammer ist in der Regel innerhalb eines Gehäuses angeordnet und zumindest abschnittsweise durch Isoliermaterial von diesem getrennt. Der Zugang zur Kammer, bei dem der Benutzer die Proben im Gehäuseinneren, insbesondere in der Kammer, lagert und wieder entnimmt, erfolgt in der Regel über eine Kammeröffnung bzw. Gehäuseöffnung, die mittels einer Gehäusetüre verschließbar ist. Ein Problem von Labortemperiervorrichtungen des Stands der Technik ist, dass sich an den Verbindungsstellen zwischen Gehäuse und Kammer, die insbesondere im Bereich der Kammeröffnung bzw. Gehäuseöffnung liegen können, Wärmebrücken ausbilden, die zu unerwünschten Störungen des Kammerklimas führen können. Bei Inkubatoren wurde beobachtet, dass sich an den Kammerinnenwänden nahe der Wärmebrücken Kondensat bildet, da an diesen Stellen Wärme lokal über die nach außen führenden Wärmebrücken abgezogen wird, was zu einer lokalen Abkühlung der Innenwände nahe der Verbindungsstellen und zur Kondensation führt. Die Bildung von Kondensat gilt es zu vermeiden, da es den Innenraum verunreinigt und Keimen als Wachstumsgrundlage dient. Zudem führen die Wärmebrücken zu einem kontinuierlichen Energieverlust. Grundsätzlich ist aber ein geringer Energieverbrauch der Labortemperiervorrichtungen anzustreben. Ähnliche Eigenschaften, also eine homogene Temperaturverteilung, eine Störungsunempfindlichkeit sowie einen geringen Energieverbrauch wünscht man sich auch bei als Kühlgeräten ausgebildeten Labortemperiervorrichtungen.

Der vorliegenden Erfindung liegt deshalb die Aufgabe zugrunde, eine verbesserte Labortemperiervorrichtung bereitzustellen, deren Kammerinnenraum von der Umgebung effizient thermisch entkoppelt ist.

Die Erfindung löst diese Aufgabe jeweils durch die Labortemperiervorrichtung gemäß Anspruch 1 und durch die Labortemperiervorrichtung gemäß Anspruch 10. Weitere technische Lösungen und bevorzugte Ausgestaltungen werden in der Beschreibung genannt, zudem sind weitere bevorzugte Ausgestaltungen Gegenstände der Unteransprüche.

Die erfindungsgemäße Labortemperiervorrichtung, die insbesondere ein Inkubator für Zellkulturen ist, dient dem temperierten Lagern von Laborproben und weist auf: ein Gehäuse mit einem von mindestens einer Gehäusewand umgebenen Gehäuseinneren, eine im Gehäuse angeordnete temperierbare Kammer mit einem von mindestens einer Kammerwand umgebenen Kammerinneren zur Aufnahme der Laborproben, mehrere Abstandselemente, wobei ein Abstandselement mindestens einen ersten Verbindungsabschnitt aufweist, mittels dem das Abstandselement und das Gehäuse verbunden sind und mindestens einen vom ersten Verbindungsabschnitt beabstandeten zweiten Verbindungsabschnitt aufweist, mittels dem das Abstandselement und die Kammer verbunden sind, so dass die Kammer mittels der Abstandselemente beabstandet zum Gehäuse gehalten wird, wobei die Abstandselemente jeweils unter Verwendung eines Materials mit einer Wärmeleitfähigkeit kleiner als 15 W/(mK) gebildet ist.

Aufgrund der geringen Wärmeleitfähigkeit der Abstandselemente wirken diese als thermische Isolatoren. Der Wärmefluss zwischen Kammer und Gehäuse wird dadurch signifikant reduziert gegenüber den herkömmlichen Lösungen, bei denen Kammer und Gehäuse durch metallische, also exzellent leitende Verbindungsmittel verbunden sind. Die Ausbildung von Wärmebrücken zwischen Kammer und Gehäuse wird somit auf ein Minimum reduziert. Da die Kammer über Abstandselemente am Gehäuse gehalten wird, kann einerseits auf weitere Verbindungselemente oder flächige Verbindungsabschnitte zwischen Kammer und Gehäuse verzichtet werden, die jeweils unerwünschte Wärmebrücke bilden würden. Andererseits wird die mechanische Positionsstabilität der Kammer im Gehäuse durch die Abstandselemente gewährleistet, deren Material sowie insbesondere deren Formgebung und Anzahl für diese Aufgabe optimierbar sind bzw. optimiert sind. Das Bereitstellen mehrerer Abstandselemente ermöglicht die Reduktion des wärmeleitenden Querschnitts der Verbindung von Kammer und Gehäuse und schafft andererseits die Möglichkeit, die mechanische Last zwischen Kammer und Gehäuse optimal zu verteilen. Weiterhin ist es möglich durch die Wahl wenigstens eines Abstandselements, das zur schwimmenden Lagerung des Abstandselements an der Kammer und/oder am Gehäuse eingerichtet ist, Relativbewegungen zwischen Kammer und Gehäuse zu ermöglichen, um so thermisch bedingte, mechanische Spannungen zu verhindern.

Durch die Verwendung der Abstandselemente mit schlechter Wärmeleitungseigenschaft wird insbesondere erreicht, dass im Falle einer als $CO_2$ Inkubator ausgebildeten Labortemperiervorrichtung eine Kondensation von Wasserdampf im Kammerinneren an jenen Stellen der Kammerwand verhindert wird, die mithilfe der Abstandselemente mit dem Gehäuse verbunden sind und in denen ein Wärmeabfluss zu einer lokalen Wärmesenke und damit zu lokalen Kondensationsherden führen würde.

Besonders bevorzugt ist ein Abstandselement unter Verwendung eines nicht-metallischen Materials gebildet. Solche nicht-metallischen Materialien weisen eine deutlich geringere Wärmeleitfähigkeit als Metalle auf. Die Wärmeleitfähigkeit des Materials, insbesondere des nicht-metallischen Materials, ist vorzugsweise kleiner als 10 W/(mK), vorzugsweise kleiner als 5 W/(mK), besonders bevorzugt kleiner als 2,5 W/(mK).

Besonders bevorzugt ist ein Abstandselement unter Verwendung eines Kunststoffs aufweisenden Materials gebildet, und besteht insbesondere aus Kunststoff. Der Kunststoff kann mit Fasern oder Füllstoffen verstärkt sein. Das Abstandselement kann aus einem Verbundmaterial bestehen. Auf Kunststoff basierende Materialien weisen eine deutlich geringere Wärmeleitfähigkeit als Metalle auf. Die Wärmeleitfähigkeit des Materials, insbesondere des kunststoff-haltigen Materials, ist vorzugsweise kleiner als 2 W/(mK), vorzugsweise kleiner als 1 W/(mK), besonders bevorzugt kleiner als 0,8 W/(mK). Besonders bevorzugt ist das Material ein Hochleistungskunststoff, der insbesondere hochtemperaturbeständig ist und insbesondere Betriebstemperaturen von 180 °C bis 200 °C toleriert. Solche Temperaturen werden in Inkubatoren während Sterilisationszyklen zur Sterilisation des Kammerinnenraums verwendet. Zudem widerstehen Hochleistungskunststoffe auch sehr tiefen Temperaturen, die in als Kühl- und Gefrierschränken ausgebildeten Labortemperiervorrichtungen vorliegen. Als besonders geeignet haben sich die Materialien Polyphenylensulfid (Kurzzeichen PPS), Polyetheretherketon (PEEK), Polyetherketon (PEK) und gefülltes Polybutylenterephthalat (PBT) erwiesen. Deren mechanische Eigenschaften lassen sich insbesondere durch Faserzusätze und Füllstoffe weiter verbessern.

Die Wärmeleitfähigkeit von Festkörpern ist grundsätzlich ein temperaturabhängiger Parameter. Im Rahmen der Beschreibung der Erfindung bezieht sich die Angabe der Wärmeleitfähigkeit standardgemäß auf eine Messung bei 20 °C. Wärmeleitfähigkeiten thermisch isolierender Materialien lassen sich insbesondere unter Verwendung des Industriestandards DIN 52612-1 bestimmen.

Vorzugsweise besteht das Abstandselement vorwiegend aus dem Material mit der Wärmeleitfähigkeit kleiner als 15 W/(mK). Vorzugsweise besteht das Abstandselement vollständig, oder im Wesentlichen vollständig, aus dem Material mit der Wärmeleitfähigkeit kleiner als 15 W/(mK).

Vorzugsweise besteht das Abstandselement zumindest zwischen dem ersten Verbindungsabschnitt und dem zweiten Verbindungsabschnitt zumindest abschnittsweise, insbesondere in einem dritten Abschnitt, aus dem Material mit der Wärmeleitfähigkeit kleiner als 15 W/(mK). Dadurch ist gewährleistet, dass der Wärmefluss im Abstandselement vollständig oder im Wesentlichen vollständig über einen Abschnitt, insbesondere den dritten Abschnitt, erfolgt, der eine Wärmeleitfähigkeit kleiner als 15 W/(mK) aufweist.

Die thermische Entkopplung zwischen dem ersten und dem zweiten Verbindungsabschnitt ist nicht nur von der Materialeigenschaft "Wärmeleitfähigkeit" abhängig, sondern auch von der geometrischen Beschaffenheit des Abstandselements. Bei einem idealen geraden Wärmeleiter bzw. thermischem Isolator mit der Querschnittsfläche A und der Länge L und der Wärmeleitfähigkeit λ ist die zeitliche Änderung des Wärme, also der durch eine Temperaturdifferenz ΔT bedingte Wärmestrom Q gegeben durch

$$\dot{Q} = \lambda \cdot \frac{A}{L} \cdot \Delta T.$$

Je länger die Strecke zwischen dem ersten und zweiten Verbindungsabschnitt ist, über die der Wärmefluss stattfindet, desto kleiner ist der Wärmestrom und desto besser ist die thermische Entkopplung, je kürzer diese Strecke ist, desto schlechter ist die thermische Entkopplung. Je kleiner der Querschnitt des Abstandselements quer zur Richtung des Wärmestroms zwischen dem ersten und zweiten Verbindungsabschnitt ist, desto kleiner ist der Wärmestrom und desto besser ist die thermische Entkopplung, je größer dieser Querschnitt ist, desto schlechter ist die thermische Entkopplung. Aufgrund der Anforderungen an die mechanische Tragfähigkeit des Abstandselements kann dieses aber nicht beliebig dünn und lang gestaltet werden. Die Lösung gemäß einer bevorzugten Ausgestaltung der Erfindung liegt darin, dass das Abstandselement -bei gegebener Größe und Form- mindestens eine Aussparung und/oder mindestens einen Hohlraum aufweist. Ein solcher Hohlraum bzw. eine solche Aussparung erlauben es, ein Abstandselement bereitzustellen, das einerseits einen geringen Wärmestrom zwischen dem ersten und dem zweiten Verbindungsabschnitt erzeugt und das andererseits eine ausreichende mechanische Belastbarkeit aufweist.

Ein erster Verbindungsabschnitt wird als solcher Bereich des Abstandselements angesehen, der in der Verbindungsposition, in der die Kammer und das Gehäuse durch das Abstandselement verbunden sind, am Gehäuse angeordnet ist und dieses insbesondere kontaktiert. Vorzugsweise ist der erste Verbindungsabschnitt am Gehäuse gelagert, insbesondere gleitend gelagert. Ein zweiter Verbindungsabschnitt wird als solcher Bereich des Abstandselements angesehen, der in der Verbindungsposition an der Kammer angeordnet ist und diese insbesondere kontaktiert. Vorzugsweise ist der zweite Verbindungsabschnitt an der Kammer gelagert, und kann auch gleitend gelagert sein.

Vorzugsweise weist ein Abstandselement mindestens einen Hohlraum und/oder mindestens eine Aussparung auf. Vorzugsweise weist das Abstandselement mindestens einen Abschnitt auf, der insbesondere als dritter Abschnitt bezeichnet wird, der den ersten und den zweiten Verbindungsabschnitt verbindet. Dieser mindestens eine dritte Abschnitt ist vorzugsweise stegförmig, wodurch der Wärmestrom reduziert wird und der thermische Widerstand groß ist. Entlang der Längsrichtung des Stegs ist dieser auf Zug oder Schub mechanisch gut belastbar. Vorzugsweise ist der stegförmige Abschnitt zwischen dem ersten und zweiten Verbindungsabschnitt linear ausgerichtet, insbesondere entlang einer virtuellen Achse, die den ersten und zweiten Verbindungsabschnitt verbindet. Dadurch ist die mechanische Belastbarkeit (Zug und/oder Schub) in axialer Richtung groß. Vorzugsweise sind mehrere dritte Abschnitte vorgesehen, die einen ersten und einen zweiten Verbindungsabschnitt verbinden.

Vorzugsweise weist der erste und/oder der zweite Verbindungsabschnitt des Abstandselements mindestens ein Loch auf, mittels dem das Abstandselement mit dem Gehäuse oder der Kammer verbindbar ist. Insbesondere kann mindestens ein als Langloch ausgebildetes Loch vorgesehen sein, das zur gleitenden Lagerung eines Verbindungselements eingerichtet sein kann, das mit dem Gehäuse oder der Kammer verbunden ist. Vorzugsweise ist ein Metallstift, insbesondere eine Schraube, vorgesehen, die sich durch das Loch oder Langloch erstreckt und die den ersten bzw. den zweiten Verbindungsabschnitt mit dem Gehäuse bzw. der Kammer verbindet.

Vorzugsweise weist das Abstandselement mehr als einen ersten und/oder zweiten Verbindungsabschnitt auf. Dadurch können die mechanischen Kräfte der Kammerbefestigung noch günstiger verteilt werden.

Vorzugsweise weist das Abstandselement einen plattenförmigen Abschnitt auf oder ist plattenförmig. Ein plattenförmiges Abstandselement ist insbesondere in Richtungen parallel der Hauptebene der Platte gut mechanisch belastbar und weist in solchen Richtungen wegen des geringen Querschnitts einen erhöhten thermischen Widerstand auf, was zur thermischen Entkopplung gewünscht ist. Zudem kann ein plattenförmiges Bauteil vorteilhaft an einer parallelen Fläche gelagert werden, insbesondere an einer Kammerwand oder Gehäusewand, die dann die Lage des plattenförmigen Bauteils stabilisiert.

Vorzugsweise weist das Abstandselement mehrere, insbesondere miteinander verbundene Stegabschnitte auf, die insbesondere zumindest teilweise in Richtung eines Verbindungsabschnitts ausgerichtet sind, an dem die Kammer mit dem Abstandselement verbunden ist.

Vorzugsweise weist das Abstandselement eine oder mehrere Aussparungen, Öffnungen oder Hohlräume auf und/oder ist zumindest abschnittsweise porös.

Vorzugsweise weist die Labortemperiervorrichtung mindestens ein Abstandselement auf, das mit einer Frontwand des Gehäuses und einer Frontwand der Kammer verbunden ist. Vorzugsweise weist die Labortemperiervorrichtung mehrere Abstandselemente auf, die im unteren Bereich der Kammer mit dem Gehäuse, insbesondere einer Frontwand des Gehäuses, und der Kammer, insbesondere einer Frontwand der Kammer, verbunden sind. Der untere Bereich ist insbesondere eine bodenseitige Frontwand der Kammer, die mittels der Abstandselemente mit einer bodenseitigen Frontwand des Gehäuses verbunden wird. "Bodenseitig" bedeutet "nahe der Bodenwand", die Bodenwand ist eine untere Außenwand der Kammer oder des Gehäuses.

Vorzugsweise weist die Labortemperiervorrichtung mehrere Abstandselemente auf, die im oberen Bereich der Kammer mit dem Gehäuse, insbesondere einer Frontwand des Gehäuses, und der Kammer, insbesondere einer Frontwand der Kammer, verbunden sind. Der obere Bereich ist insbesondere eine deckenseitige Frontwand der Kammer, die mittels der Abstandselemente mit einer deckenseitigen Frontwand des Gehäuses verbunden wird. "Deckenseitig" bedeutet "nahe der Deckenwand", die Deckenwand ist eine obere Außenwand der Kammer oder des Gehäuses.

Vorzugsweise ist mindestens ein Abstandselement vorgesehen, das zur schwimmenden Lagerung des Abstandselements an der Kammer und/oder am Gehäuse eingerichtet ist. Die schwimmende Lagerung erlaubt eine Relativbewegung zwischen Kammer und Gehäuse, die zum Verhindern von thermisch bedingten, mechanischen Spannungen in der Labortemperiervorrichtung verwendbar ist. Die schwimmende Lagerung gelingt insbesondere über eine Gleitlagerung des entsprechenden ersten und/oder zweiten Verbindungsabschnitts des Abstandselements. Die Gleitlagerung gelingt insbesondere, indem der entsprechende Verbindungsabschnitt ein Langloch aufweist, in dem ein Gleitelement der Kammer oder des Gehäuses gleitet und gleichzeitig die Verbindung bewirkt.

Gemäß einem zweiten besonderen Aspekt der Erfindung ist eine Labortemperiervorrichtung gestaltet, wie folgt:

Eine erfindungsgemäße Labortemperiervorrichtung, insbesondere Inkubator für Zellkulturen, weist auf:

ein Gehäuse mit einer Außentüre und einer durch die Außentüre verschließbaren Gehäuseöffnung, die in einer Gehäusefrontwand ausgebildet ist,

eine im Gehäuse angeordnete, ein Kammerinneres umgebende Kammer mit einer Kammeröffnung, die in einer Kammerfrontwand ausgebildet ist,

wobei die Gehäusefrontwand und die Kammerfrontwand durch eine erste Dichtung voneinander getrennt sind, die um die Kammeröffnung umläuft,

wobei die Außentüre eine Innenseite mit einer zweiten Dichtung aufweist, die im geschlossenen Zustand der Außentüre an der ersten Dichtung anliegt und um die Kammeröffnung umläuft.

Eine solche erfindungsgemäße Labortemperiervorrichtung bietet den Vorteil, dass der bei geschlossener Außentüre zwischen Kammertüre und Gehäusetüre gebildete Zwischenraum nicht mit dem Gehäuse in Kontakt steht. Dies ist darin begründet, dass die zweite Dichtung im geschlossenen Zustand der Außentüre durch das lückenlose Kontaktieren ersten Dichtung den Zwischenraum seitlich begrenzt. Jeglicher Kontakt des im Zwischenraum enthaltenen, von der Kammer erwärmten Luftgemisches mit der nicht-temperierten Außenwand des Gehäuses und somit jeglicher konvektionsbedingte Wärmetransport zwischen dem Zwischenraum und dem Gehäuse ist somit unmöglich.

Die erste Dichtung und die zweite Dichtung sind vorzugsweise aus einem elastomeren Material gefertigt, welches hochtemperaturbeständig ist, insbesondere bis 200 °C. Das elastomere Material ist insbesondere ein elastomerer Kunststoff, insbesondere ein Silikon-Kunststoff, insbesondere ein Kunststoffschaum, vorzugsweise ein Silikonschaum.

Die erste Dichtung verbindet die Gehäusefrontwand und die Kammerfrontwand. Die erste Dichtung weist eine minimale oder durchschnittliche Materialdicke auf, die, gemessen senkrecht zur Gehäusefrontwand, geringer ist als diese kürzeste Verbindung bzw. die Spaltbreite zwischen der Gehäusefrontwand und der Kammerfrontwand. Insbesondere ist die minimale oder durchschnittliche Materialdicke d2 der ersten Dichtung weniger als 1 cm, insbesondere 0,8 cm, vorzugsweise zwischen 0,2 cm und 1 cm. Die kürzeste Verbindung wird insbesondere durch die Spaltbreite zwischen der Gehäusefrontwand und der Kammerfrontwand gegeben. Die Spaltbreite d1 liegt vorzugsweise bei mehr als 1,0 cm, und liegt vorzugsweise im Bereich von mehr als 1,2 cm, vorzugsweise im Bereich 1,0 cm bis 2,0 cm, vorzugsweise zwischen 1,2 cm und 1,8 cm, vorzugsweise zwischen 1,2 und 1,6 cm. Der Spalt kann aber auch breiter sein, somit kann auch die erste Dichtung breiter sein. Durch die genannten bevorzugten Ausgestaltungen (maximale Wärmestromstrecke in Richtung der Spaltbreite und minimaler wärmeleitender Querschnittsenkrecht zu dieser Richtung) ist der thermische Widerstand der ersten Dichtung maximal.

Die zweite Dichtung weist vorzugsweise einen näher an der Außentüre gelegenen, umlaufenden ersten Dichtungsbereich auf, der ein höheres Elastizitätsmodul aufweist als ein zweiter, vorzugsweise integral mit dem ersten Dichtungsbereich verbundener zweiter Dichtungsbereich, der insbesondere weicher ist und der in der geschlossenen Position der Außentüre näher an der ersten Dichtung liegt und diese kontaktiert. Durch einen weicheren Dichtungsbereich zwischen Außentüre und Gehäuse lässt sich der Zwischenraum zwischen Außentüre und Kammertüre besser abdichten, zudem werden die zum Schließen der Kammertüre erforderlichen Kräfte reduziert, so dass die Bedienung für den Benutzer leichter wird. Der erste Dichtungsbereich ist vorzugsweise unter Verwendung eines Elastomers, insbesondere Silikon, gefertigt, der zweite Dichtungsbereich vorzugsweise unter Verwendung eines geschäumten Elastomers, insbesondere Silikonschaums.

Gemäß einem dritten besonderen Aspekt der Erfindung ist eine Labortemperiervorrichtung gestaltet, wie folgt:
Eine erfindungsgemäße Labortemperiervorrichtung, insbesondere Inkubator für Zellkulturen, weist auf:

ein Gehäuse mit einer Außentüre und einer durch die Außentüre verschließbaren Gehäuseöffnung, die in einer Gehäusefrontwand ausgebildet ist,

eine im Gehäuse angeordnete, ein Kammerinneres umgebende Kammer mit einer Kammeröffnung, die in einer Kammerfrontwand ausgebildet ist,

wobei die Gehäusefrontwand und die Kammerfrontwand durch einen Spalt getrennt sind, und

wobei ein um die Kammerfrontwand umlaufender Wand-Endabschnitt der Gehäusefrontwand die Gehäuseöffnung bildet, und wobei die Kammerfrontwand einen Flansch um die Kammeröffnung bildet, der in einem um die Kammeröffnung umlaufenden Wand-Endabschnitt endet,

wobei der Wand-Endabschnitt der Gehäusefrontwand und der Wand-Endabschnitt der Kammerfrontwand jeweils eine Dicke zwischen 0,5 mm und 4,0 mm, vorzugsweise zwischen 0,8 mm und 3,0 mm, aufweisen und einander gegenüberliegen, um diesen Spalt zu bilden.

Durch diese Gestaltung ist die Wärmeübertragung zwischen Wand-Endabschnitt der Gehäusefrontwand und der Wand-Endabschnitt der Kammerfrontwand mittels Wärmestrahlung minimiert und die thermische Entkopplung zwischen Kammer und Gehäuse weiter verbessert.

Insbesondere sind der Wand-Endabschnitt der Gehäusefrontwand und der Wand-Endabschnitt der Kammerfrontwand in Bezug auf eine Projektionsrichtung senkrecht zur Ebene der Kammeröffnung überlappungsfrei angeordnet, das heißt, das die Projektionen der Wand-Endabschnitte auf diese Ebene einander nicht schneiden. Der insbesondere planare Wand-Endabschnitt der Gehäusefrontwand und der insbesondere planare Wand-Endabschnitt der Kammerfrontwand liegen vorzugsweise in derselben Ebene.

Die Labortemperiervorrichtung zum Lagern von Laborproben ist insbesondere ein Temperierschrank zum Temperieren von Laborproben. Solche Geräte werden elektrisch betrieben und weisen einen Spannungsanschluss auf.

Der Temperierschrank temperiert die Laborproben, das heißt, er hält das Gehäuseinnere und damit die dort lagernden Laborproben im Rahmen von Toleranzen durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einer als Klimaschrank ausgebildeten Laborschrankvorrichtung wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine CO2, O2 und/oder N2-Konzentration. Ein solcher Klimaschrank ist beispielsweise ein Inkubator für aus lebenden Zellkulturen bestehende Laborproben.

Die Labortemperiervorrichtung weist vorzugsweise ein Gehäuse auf. Das Gehäuse ist vorzugsweise ein äußeres Gehäuse, dessen Gehäusewände mit der Umgebung in Kontakt stehen. Die Gehäusetüre kann entsprechend eine äußere Gehäusetüre sein, die in der Verschlussposition an die Umgebung grenzt.

Die Gehäusetüre weist insbesondere eine Scharniereinrichtung auf, welche die Gehäusetüre schwenkbar mit dem Gehäuse verbindet. Eine solche Schwenktüre wird durch eine Rotation zwischen einer geöffneten Position und der Verschlussposition bewegt. Die Scharniereinrichtung kann insbesondere an der -im bestimmungsgemäßen Gebrauch der Laborschrankvorrichtung - vertikal orientierten Außenkante eines quaderförmigen Gehäuses liegen, welche an die Gehäuseöffnung angrenzt. Die Bodenplatte eines quaderförmigen Gehäuses ist im bestimmungsgemäßen Gebrauch der Laborschrankvorrichtung horizontal angeordnet, die Seitenwände des Gehäuses sind insbesondere vertikal angeordnet, und die Deckplatte des Gehäuses ist insbesondere der Bodenplatte gegenüberliegend horizontal angeordnet.

Die Kammertüre oder Gehäusetüre kann aber auch eine Schiebetüre sein, die durch eine translatorische Bewegung zwischen einer geöffneten Position und der Verschlussposition bewegt wird. Auch eine gemischt schwenkende/translatorische Bewegung der Kammertüre oder Gehäusetüre ist möglich.

Eine Datenverarbeitungseinrichtung ist vorzugsweise Bestandteil der elektrischen Steuereinrichtung, die Funktionen der Labortemperiervorrichtung steuert. Die Funktionen der Steuereinrichtung sind insbesondere durch elektronische Schaltkreise implementiert. Die Steuereinrichtung kann ein Recheneinheit (CPU) zum Verarbeiten von Daten und/oder einen Mikroprozessor aufweisen, der die Datenverarbeitungseinrichtung beinhalten kann. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Die Funktionen des Inkubators und/oder der Steuereinrichtung können in Verfahrensschritten beschrieben werden. Sie können als Bestandteile des Steuerungsprogramms realisiert sein, insbesondere als Unterprogramme des Steuerungsprogramms.

Vorzugsweise ist die Labortemperiervorrichtung ein Labortemperierschrank, insbesondere ein Inkubator. Der Inkubator ist ein Labor-Inkubator und damit ein Gerät, mit dem kontrollierte Klimabedingungen für verschiedene biologische Entwicklungs- und Wachstumsprozesse geschaffen und erhalten werden können. Er dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine als Heiz- und/oder Kühleinrichtung ausgeführte Temperiereinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des $CO_2$ und/oder des $O_2$ und/oder des $N_2$-Gehalts Gehalts des Gases und/oder eine Einstelleinrichtung zur Einstellung der Luftfeuchtigkeit in der Inkubatorkammer des Inkubators.

Der Inkubator weist insbesondere die Inkubatorkammer (=Kammer) auf, ferner vorzugsweise eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden, obwohl die Luftfeuchtigkeit selber nicht durch einen Luftfeuchte-Sensor (rH-Sensor) gemessen wird und die Luftfeuchtigkeit nicht Eingangsgröße des Regelkreises ist. Eine mit Wasser gefüllte Wanne in der Inkubatorkammer kann geheizt oder gekühlt werden, um über die Verdunstung die Luftfeuchtigkeit einzustellen. $CO_2$-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Die Steuerungseinrichtung kann dazu eingerichtet sein, dass ein Programmparameter oder ein Steuerungsparameter des Inkubators automatisch in Abhängigkeit von anderen Daten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, die relative Gaskonzentration von $O_2$ und/oder $CO_2$ und/oder $N_2$ im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen.

Die Labortemperiervorrichtung bzw. der Inkubator kann genau eine Kammer aufweisen, kann aber auch mehrere Kammern aufweisen, deren Atmosphäre (Temperatur, relative Gaskonzentration, Luftfeuchte) insbesondere individuell oder gesammelt einstellbar sein kann. Eine typische Größe des Inneren einer Kammer liegt zwischen 50 und 400 Litern, wobei auch für besondere Anwendungen (IVF) kleinere Kammergrößen möglich sind, insbesondere 10 bis 49 Liter.

Die im Rahmen der Erfindung genannten Merkmale und bevorzugten Ausgestaltungen der erfindungsgemäßen Labortemperiervorrichtung gemäß Anspruch 1 können auch zur Ausgestaltung einer erfindungsgemäßen Labortemperiervorrichtung gemäß dem zweiten oder dritten besonderen Aspekt verwendet werden. Auch die Labortemperiervorrichtung gemäß Anspruch 1 kann durch Merkmale der erfindungsgemäßen Labortemperiervorrichtung gemäß dem zweiten oder dritten besonderen Aspekt ausgestaltet werden. Weitere bevorzugte Ausgestaltungen der erfindungsgemäßen Labortemperiervorrichtung lassen sich der Beschreibung der Ausführungsbeispiele gemäß den Figuren entnehmen.

Es zeigen:

Fig. 1a zeigt eine perspektivische seitlich-frontale Ansicht eines erfindungsgemäßen Inkubators gemäß Ausführungsbeispiel, im geschlossenen Zustand der Gehäusetüre.

Fig. 1b zeigt eine perspektivische seitlich-rückwärtige Ansicht des Inkubators der Fig. 1a.

Fig. 1c zeigt eine perspektivische seitlich-frontale Ansicht des Inkubators der Fig. 1a, im geöffneten Zustand der Gehäusetüre.

Fig. 2a zeigt eine perspektivische seitlich-frontale Ansicht des Inkubators der Fig. 1a, mit ausgeblendeter Gehäusetüre und in einem Querschnitt entlang einer Ebene, die parallel zur Seitenwand und zentral durch den Inkubator verläuft.

Fig. 2b zeigt als Detail der Fig. 2a einen der als Abstandselemente zwischen Kammer und Gehäuse dienenden Zugangsports des Inkubators.

Fig. 3a zeigt eine perspektivische Rückansicht der Gehäusefrontwand und der Kammer, der zwischen der Gehäusefrontwand und dem Kammerfrontblech angeordneten Abstandselemente und der an der Kammerrückwand angeordneten Zugangsports des Inkubators der Fig. 1a.

Fig. 3b zeigt eine perspektivische seitlich-rückwärtige Ansicht der in Fig. 3a gezeigten Teile.

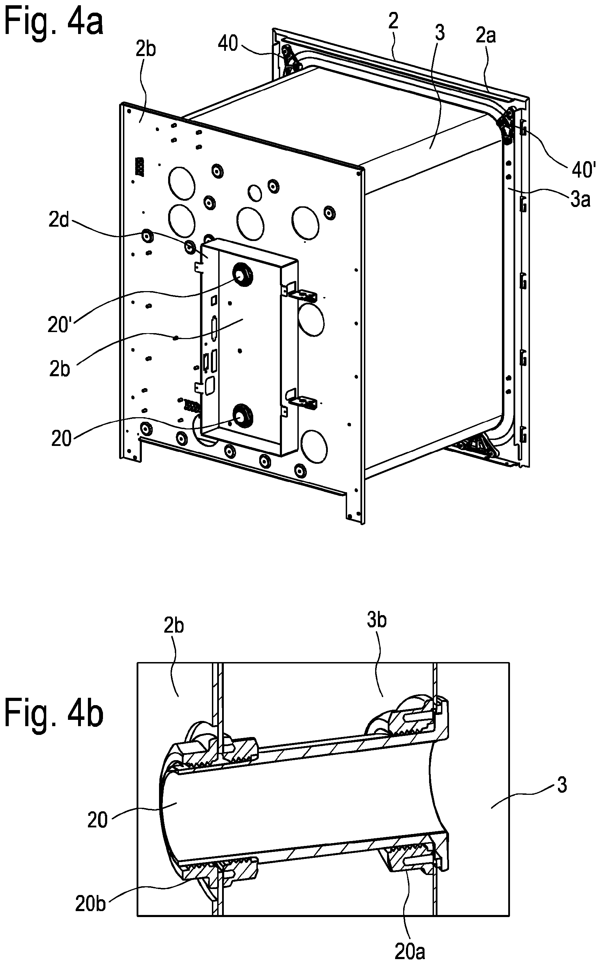

Fig. 4a zeigt eine perspektivische seitlich-rückwärtige Ansicht der in Fig. 3a gezeigten Teile sowie einen Teil der Gehäuserückwand mit Anbauten.

Fig. 4b zeigt eine Querschnittsansicht eines als Abstandselement dienenden Zugangsports entsprechend der Anordnung in Fig. 4a.

Fig. 5a zeigt einen Ausschnitt einer perspektivischen Rückansicht der Gehäusefrontwand und der Kammer im unteren Bereich des Inkubators der Fig. 3a und der dort zwischen der Gehäusefrontwand und dem Kammerfrontblech angeordneten Abstandselemente.

Fig. 5b zeigt als einen Ausschnitt der Fig. 5a das dort zentral angebrachte Abstandselement.

Fig. 5c zeigt eine perspektivische Querschnittsansicht des kammerseitigen zweiten Verbindungsabschnitts des Abstandselements der Fig. 5b, wobei der Querschnitt senkrecht zur Hauptebene des plattenförmigen Abstandselements und entlang der Linie L2 der Fig. 5b verläuft.

Fig. 5d zeigt eine perspektivische Querschnittsansicht des gehäuseseitigen ersten Verbindungsabschnitts des Abstandselements der Fig. 5b, wobei der Querschnitt senkrecht zur Hauptebene des plattenförmigen Abstandselements und entlang der Linie L1 der Fig. 5b verläuft.

Fig. 5e zeigt als einen Ausschnitt der Fig. 5a das dort links im Bild gezeigte Abstandselement.

Fig. 5f zeigt eine perspektivische Querschnittsansicht des gehäuseseitigen ersten Verbindungsabschnitts des Abstandselements der Fig. 5e, wobei der Querschnitt senkrecht zur Hauptebene des plattenförmigen Abstandselements und entlang der Linie L3 der Fig. 5e verläuft.

Fig. 5g zeigt eine Aufsicht auf das Abstandselement der Fig. 5e.

Fig. 5h zeigt eine perspektivische Ansicht mit Blick auf die auch in Fig. 5e gezeigte Hauptseite des plattenförmigen Abstandelements.

Fig. 5i zeigt eine perspektivische Ansicht mit Blick auf die auch in Fig. 5h abgwandte und dort nicht sichtbare Hauptseite des plattenförmigen Abstandelements, die im montierten Zustand zur Frontseite des Inkubators gewandt ist.

Fig. 6a zeigt einen Ausschnitt einer perspektivischen Rückansicht der Gehäusefrontwand und der Kammer im oberen Bereich des Inkubators der Fig. 3a und der dort zwischen der Gehäusefrontwand und dem Kammerfrontblech angeordneten Abstandselemente.

Fig. 6b zeigt als einen Ausschnitt der Fig. 6a das dort links im Bild sichtbare Abstandselement.

Fig. 6c zeigt eine perspektivische Querschnittsansicht der kammerseitigen zweiten Verbindungsabschnitte des Abstandselements der Fig. 6b, wobei der Querschnitt senkrecht zur Hauptebene des plattenförmigen Abstandselements und entlang der Linie L4 der Fig. 6b verläuft.

Fig. 6d zeigt eine perspektivische Querschnittsansicht durch den gehäuseseitigen ersten Verbindungsabschnitt des Abstandselements der Fig. 6b, wobei der Querschnitt senkrecht zur Hauptebene des plattenförmigen Abstandselements und entlang der Linie L5 der Fig. 6b verläuft.

Fig. 6e zeigt eine Aufsicht auf das Abstandselement der Fig. 6b.

Fig. 6f zeigt eine perspektivische Ansicht mit Blick auf die auch in Fig. 6b gezeigte Hauptseite des plattenförmigen Abstandelements.

Fig. 6g zeigt eine perspektivische Ansicht mit schrägem Blick auf die auch in Fig. 6f abgwandte und dort nicht sichtbare Hauptseite des plattenförmigen Abstandelements, die im montierten Zustand zur Frontseite des Inkubators gewandt ist.

Fig. 7 zeigt ausschnittsweise eine seitliche Querschnittsansicht des in Fig. 1a gezeigten Inkubators, wobei der Querschnitt parallel zu einer Seitenwand des Inkubators verläuft und einen oberen Frontbereich des Inkubators zeigt.

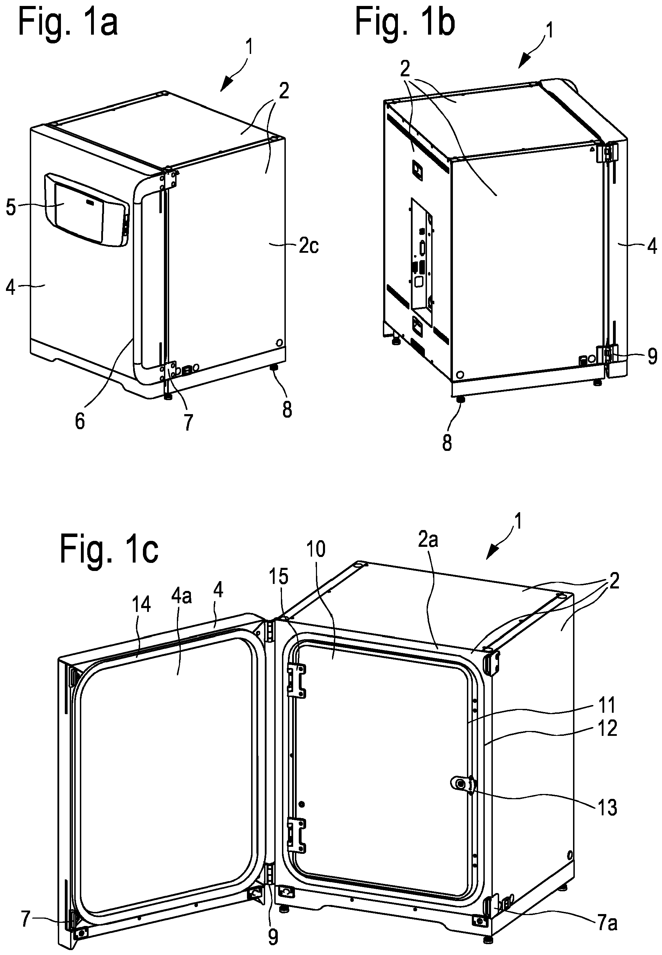

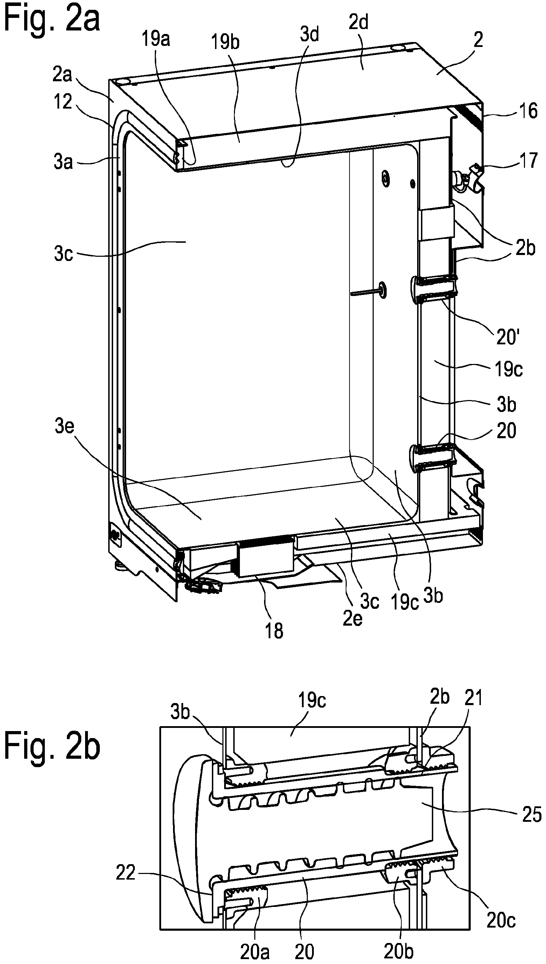

Fig. 1a zeigt die als Inkubator 1 ausgebildete Labortemperiereinrichtung 1 für das Wachstum von Zellkulturen, hier ein als CO2-Inkubator ausgebildeter Labortemoerierschrank für das Wachstum von eukaryotischen Zellen. Der Inkubator 1 hat ein Gehäuse 2 mit einem von mindestens einer Gehäusewand 2 umgebenen Gehäuseinneren und eine im Gehäuse angeordnete temperierbare Kammer 3 (siehe Fig. 2a) mit einem von mindestens einer Kammerwand umgebenen Kammerinneren zur Aufnahme der Laborproben. Die Außenwände des Gehäuses sind so miteinander verbunden, dass sie alle weiteren Bestandteile des Inkubators tragen. Das Gehäuse ruht auf Sockeln 8. Im bestimmungsgemäßen Gebrauch sind die Außenseiten der Seitenwände 2c des Gehäuses, die Frontwand 2a, die Rückwand 2b sowie die Außenseite der Gehäusetüre 4 und deren Innenseite 4a, sowie die Seitenwände der Kammer, der Kammerfrontwand 3a, und die Kammerrückwand 3b vertikal, also parallel zur Gravitationsrichtung angeordnet. Die obere Außenseite 2d und die nicht sichtbare Bodenseite des Gehäuses und die Bodenwand und Deckenwand der Kammer sind entsprechend horizontal angeordnet. Als Richtung "nach unten" ist im Kontext der Beschreibung der Erfindung immer die Richtung der Gravitation gemeint, anhand der eine bestimmungsgemäß betriebener Labortemperiervorrichtung ausgerichtet ist, die Richtung "nach oben" ist die entgegengesetzte Richtung. Die Richtung "nach vorne" bezeichnet die horizontale Richtung zur Vorderseite der geschlossenen Gehäusetüre, die Richtung "nach hinten" bezeichnet die horizontale Richtung zur Rückseite des Inkubators. Die Kammer ist aus Edelstahl gefertigt, das Gehäuse aus lackiertem Metallblech.

Die Gehäusetüre 4 trägt eine Benutzerschnittstelleneinrichtung 5, die hier ein berührungsempfindliches Display umfasst, das vom Benutzer zum Lesen und Eingeben von Informationen verwendet wird. Die Gehäusetüre weist zwei Scharniere 9 auf, die die Gehäusetüre mit dem Gehäuse 2 verbinden. Mittels einer magnetischen Verriegelungseinrichtung 7, die einen oberen und unteren gehäuseseitigen Halteabschnitt 7a und einen einen oberen und unteren gehäusetüreseitigen Halteabschnitt 7b beinhaltet, wird die Gehäusetüre in der geschlossenen Position gehalten. Die Gehäusetüre weist einen Türgriff 6 auf, der an den Positionen des oberen und unteren gehäusetüreseitigen Halteabschnitts 7b mit der Gehäusetüre verbunden ist und der sich vertikal erstreckt.

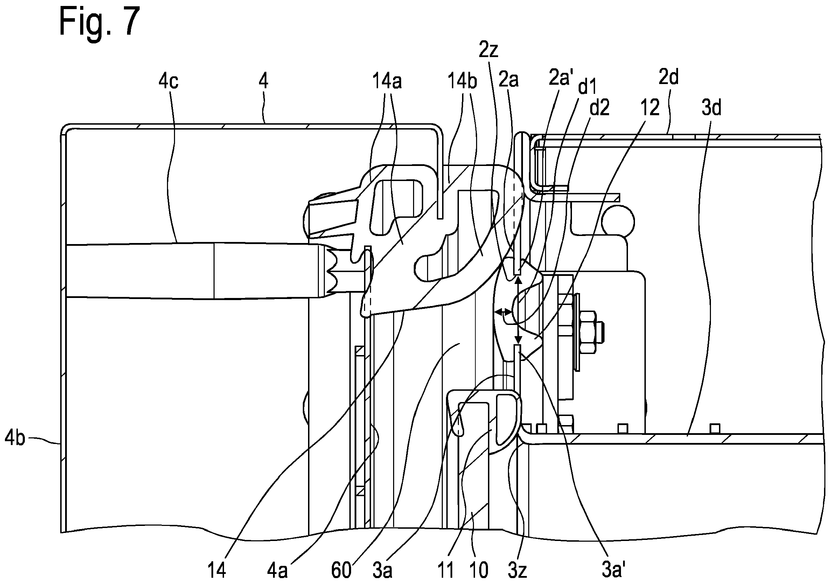

Die Gehäusefrontwand 2a verläuft vertikal und fluchtet mit der auch vertikal verlaufenden Kammerfrontwand 3a, d.h. die nach vorne weisenden Flächen, und hier auch die nach hinten weisenden Flächen, der Gehäusefrontwand 2a und der Kammerfrontwand 3a liegen im Wesentlichen in derselben Ebene, siehe Fig. 7.

Wie in Fig. 1c gezeigt ist, ist zwischen der Gehäusefrontwand 2a und der Kammerfrontwand 3a eine elastische Dichtung 12 eingebracht, die dort mithilfe von Nuten formschlüssig gehalten wird, in die die Ränder der Gehäusefrontwand 2a und der Kammerfrontwand 3a eingreifen, siehe auch Fig. 7.

In Fig. 1c ist die Gehäusetüre 4 geöffnet gezeigt. Die Kammertüre 10 ist mittels der Scharniere 15 an der Kammerfrontwand 3a befestigt, und wird in der gezeigten Position von einem magnetischen Handverschluss 13 geschlossen gehalten, so dass das Kammerinnere nicht zugänglich ist. Aufgrund der Transparenz der Kammertüre 10 ist das Innere für den Benutzer in dieser Position aber sichtbar. Die Kammertüre wird durch eine umlaufende elastische Dichtung 11 der Kammertüre gasdicht an der Kammerfrontwand gehalten. Die Innenseite 4a der Gehäusetüre weist eine umlaufende elastische Dichtung 14 auf, die im geschlossenen Zustand der Gehäusetüre an der Gehäusefrontwand und der dort umlaufenden Dichtung 12 bündig anliegt und eine gasdichte Abschirmung des Bereichs zwischen Kammertüre 10 und Gehäusetüre 4a erzielt.

Wie in Fig. 2a teilweise sichtbar ist, weist der Inkubator zwei Temperiereinrichtungen auf, die den Kammerinnenraum 3 temperieren, d.h. deren Temperatur durch eine Temperaturregelung einstellen. Ein Teil der dazu notwendigen Bauteile 18 sind zwischen der Gehäusebodenwand 2e und der Kammerbodenwand 3e angeordnet. Die Heizwendel eines oberen Heizkreises (nicht gezeigt) sind mit der Außenseite der Kammerdeckenwand 3d und einem oberen Bereich der Kammerseitenwände, hier etwa dem oberen 2/3 entlang der Höhe der Seitenwände 3c der Kammer, thermisch gekoppelt und verbunden. Die Heizwendel eines unteren Heizkreises (nicht gezeigt) sind mit der Außenseite der Kammerbodenwand 3e und einem unteren Bereich der Kammerseitenwände, hier etwa dem unteren 1/3 entlang der Höhe der Seitenwände 3c der Kammer, thermisch gekoppelt und verbunden.

Eine thermische Isoliereinrichtung 19 ist zwischen Kammer und Gehäuse vorgesehen. Sie isoliert die Kammer, mit daran anliegenden Temperiereinrichtungen von dem Gehäuse, das an seiner Außenseite direkt mit der Umgebung in Verbindung steht. Der Inkubator arbeitet normalerweise bei Außentemperaturen zwischen 18 °C und 28 °C. Die Temperiereinrichtungen bzw. die Temperaturregelung arbeitet in diesem Bereich besonders effizient. Die Isoliereinrichtung umfasst ein U-förmig gebogenes Isolierelement 19b aus Glaswolle bzw. Mineralwolle, das die Kammerdeckenplatte und die beiden Kammerseitenwände 3c umgibt. Er mündet zum Boden und an die Rückwand hin an Isolierplatten 19c aus PIR-Schaum (Polyisocyanurat-Schaum), und wird zur Frontseite von Gehäuse und Kammer durch einen umlaufenden Nadelvliesstreifen 19a abgeschlossen, der an der Innenseite der Gehäusefrontwand 2a, der Kammerfrontwand 3a und der Dichtung 12 anliegt. Die thermische Isolierung der Kammer nach außen wird durch die erfindungsgemäßen Maßnahmen optimiert.

An der Gehäuserückwand 2b ist eine doppelte Gehäuserückwand 16 zur Abdeckung rückseitig angebrachter Komponenten befestigt. Die Rückwand ist mittels eines Griffs 17 abnehmbar.

Wie in Fig. 2b im Detail gezeigt ist, weist der Inkubator an seiner Rückseite zwei Zugangsports 20, 20' auf, die dem Benutzer ermöglichen, durch Öffnungen 20h, 20'h in der Rückwand der Kammer Kabel ins Innere der Kammer zu verlegen, beispielsweise um im Inneren angeordnete Messgeräte anzusteuern. Ein Port 20, 20' dient vorliegend auch als Abstandselement, der die Kammer 3 gegenüber dem Gehäuse 2 in einem Abstand hält und einen Teil des Gewichts der Kammer und deren Anbauten und Inhalte abstützt. Dazu weist der Zugangsport ein zylinderförmiges Bauteil 20 auf, das aus einem Material mit geringer Wärmeleitfähigkeit kleiner als 15 W/(mK) besteht, hier PPS. Diese weist einen als zweiter Verbindungsabschnitt dienenden Flansch 22 auf, der an der Innenseite der Kammerrückwand 3b anliegt und mittels eines ersten Gewinderings 20a aus PPS gegen die Kammerrückwand 3b gedrückt wird. Das nach außen weisende Ende 21 des zylinderförmigen Bauteils 20 dient als erster Verbindungsabschnitt, mittels dem das Abstandselement 20 am Gehäuse befestigt ist. Dazu werden ein dritter Gewindering 20b und ein zweiter Gewindering 20b auf das zylinderförmige Bauteil 20 geschraubt, so dass die Gehäuserückwand 2b zwischen dem zweiten und dritten Gewindering fixiert wird. Für die Befestigung der Gewinderinge 20a, 20b, 20c weist das zylinderförmige Bauteil 20 entsprechende Außengewinde auf. Das Abstandselement 20' ist analog aufgebaut. Wenn der Zugangsport nicht benötigt wird, ist er von einem Stopfen 25 aus thermisch isolierendem Material, z.B. Silikonschaum, gefüllt.

Erfindungsgemäß wird die Kammer 3 durch mehrere Abstandselemente in Distanz zum Gehäuse gehalten, die eine Wärmeleitfähigkeit kleiner als 15 W/(mK) aufweisen, hier ca. jeweils 0,5 W/(mK) durch die Verwendung eines PPS, das mit Faserfüllstoffen verstärkt ist. Der Inkubator hat mehrere Abstandselemente 30, 30', 30", 40, 40', 20, 20', wobei ein Abstandselement mindestens einen ersten Verbindungsabschnitt 31, 41, 21 aufweist, mittels dem das Abstandselement und das Gehäuse 2 verbunden sind und mindestens einen vom ersten Verbindungsabschnitt beabstandeten zweiten Verbindungsabschnitt 32, 42, 22 aufweist, mittels dem das Abstandselement und die Kammer verbunden sind.

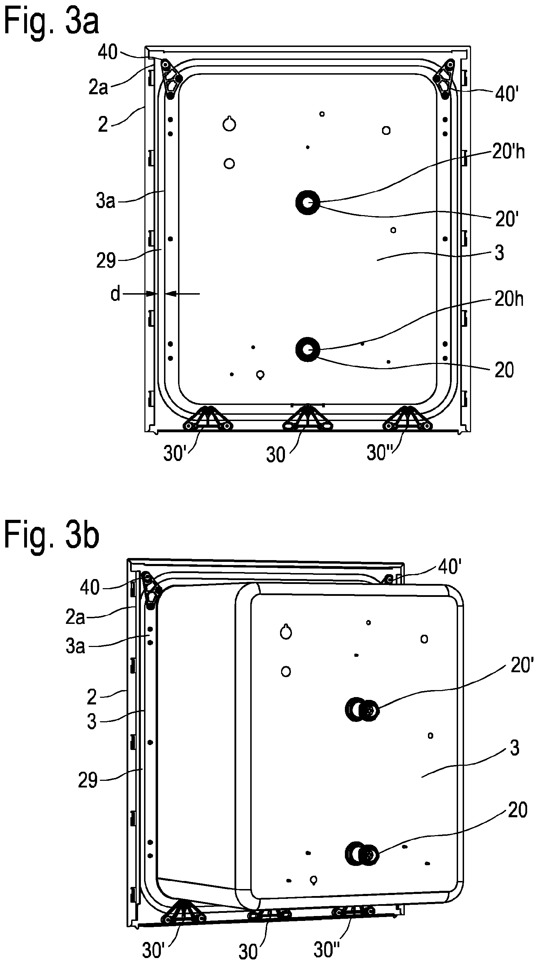

Fig. 3a zeigt eine perspektivische Rückansicht der Gehäusefrontwand 2a und der Kammer 3, der zwischen der Gehäusefrontwand 2a und der Kammerfrontwand 3a angeordneten Abstandselemente 30, 30', 30", 40, 40' und der an der Kammerrückwand 3b angeordneten, auch als Abstandselemente dienenden Zugangsports 20, 20' des Inkubators der Fig. 1a.

Durch die frontseitig vorgesehenen Abstandselemente 30, 30', 30", 40, 40' wird die Kammerfrontwand 3a, die mit der Gehäusefrontwand 2a fluchtet, in einem Abstand d gehalten, der konstant ca. 14 mm beträgt. Dies resultiert in einem um die Kammeröffnung umlaufenden Spalt 29, der von der thermisch isolierenden Dichtung 12 gefüllt wird. Die Abstandselemente 30, 30', 30", 40, 40' sind erfindungsgemäß so gebildet, dass sie einerseits den Hauptteil des Gewichts der Kammer, sowie deren Anbauten und deren maximal zulässiges Füllgewicht über die gesamte Lebensdauer des Inkubators problemlos und zuverlässig tragen können. Die Anzahl der frontseitigen Abstandelemente ergibt sich aus der Schnittmenge der Anforderungen an die mechanische, chemische und thermische Belastbarkeit der Verbindung und an die thermische Isolierfähigkeit. Diese Parameter können einerseits durch die geeignete Materialauswahl und andererseits durch die vorteilhafte geometrische Konstruktion der Abstandelemente beeinflusst werden.

Als Material der Abstandelemente, aus dem diese insbesondere durch ein Spritzgussverfahren integral hergestellt wurden, wurde hier ein Hochleistungskunststoff gewählt, insbesondere ein Verbundstoff mit einer Matrix aus Hochleistungskunststoff. Dieser wurde vorliegend als PPS GF 40 gewählt, also ein PPS mit 40% Zusatz von Glasfasern, das hier mit einem weiteren Zusatz von 25% an mineralischen Füllstoffen versehen wurde. Daraus resultiert eine hervorragende thermische Belastbarkeit von bis zu 220 °C. Dies erlaubt es problemlos, die Kammer zu Sterilisationszwecken auf 180 °C aufzuheizen, was eine Standardanforderung an moderne Inkubatoren ist. Die thermische Ausdehnung des genannte PPS-Materials bei 20 °C, gemessen insbesondere zwischen 20 °C und 60 °C, liegt bei $15*10^{-6}$ $K^{-1}$ in Längsrichtung zur Glasfaser, und bei $30*10^{-6}$ $K^{-1}$ in Querrichtung zur Glasfaser. Die Wärmeleitfähigkeit des PPS-Materials beträgt nur 0,5 W/(mK), somit ergibt sich ein hoher thermischer Widerstand, ideal für die thermische Entkopplung von Kammer und Gehäuse. Die Zugfestigkeit des PPS-Materials gemäß ISO 527 beträgt ca. 150 MPa.

Die geometrische Struktur der frontseitigen Abstandelemente wurde hinsichtlich der aufzunehmenden Traglast und zur Reduzierung des Wärmestroms optimiert. Dazu wurde insbesondere der den Wärmestrom bestimmende Querschnitt durch den zwischen dem ersten und zweiten Verbindungsabschnitt des Abstandelements 30 gelegenen "dritten Abschnitt" minimiert, andererseits wurde die vom Wärmestrom zu bewältigende Distanz durch Maximierung der Länge des dritten Abschnitts angestrebt. Dazu wurden hier die bodenseitigen Abstandelemente 30, 30', 30" so konstruiert, dass diese -jeweils baugleich- mehrere stegförmige Abschnitte 35a, 35b, 35c, 35d, 35e aufweisen, welche den ersten Verbindungsabschnitt 31 mit dem zweiten Verbindungsabschnitt 32 verbinden.

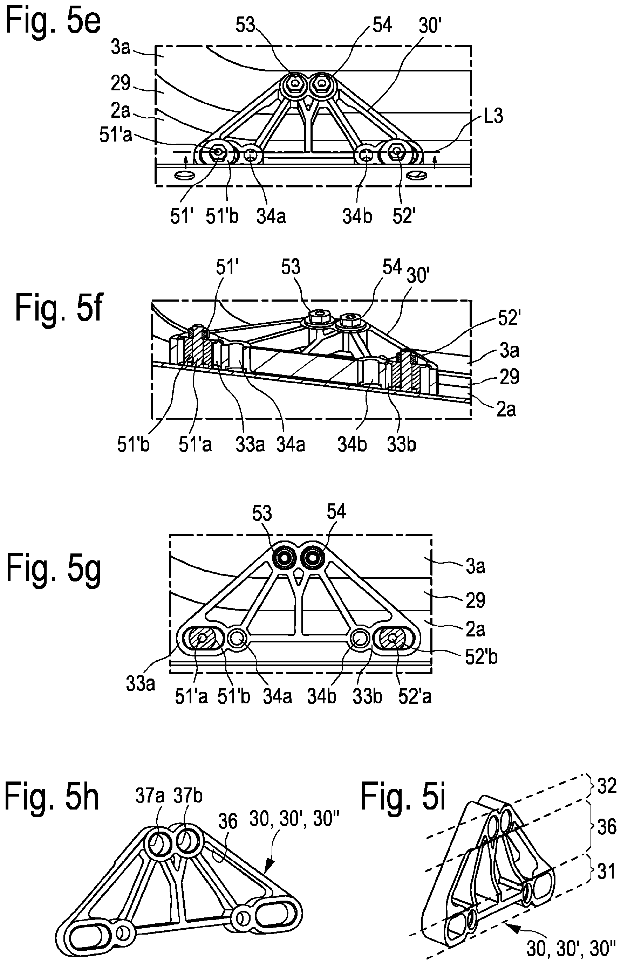

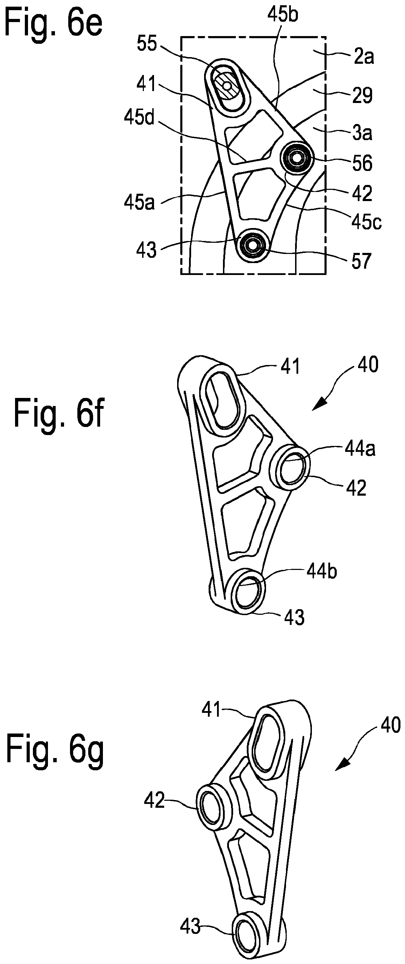

Ein frontseitiges Abstandelement 30, 30', 30", 40, 40' ist vorliegend ein im Wesentlichen plattenförmiges Bauteil, das zwei gegenüberliegende Hauptseiten aufweist, nämlich die in Fig. 5h bzw. in Fig. 6f sichtbare Rückseite des Abstandelements und die in Fig. 5i bzw. in Fig. 6g sichtbare Vorderseite des Abstandelements, die sich parallel zur Hauptebene des plattenförmigen Bauteils erstrecken. Die Ansichten in Fig. 5g und 6e sind parallel dieser Hauptebene. Der Querschnitt der frontseitigen plattenförmigen Abstandelemente parallel zu dessen Hauptebene folgt im Wesentlichen einer dreieckförmigen Kontur, im Falle der bodenseitigen Abstandselemente gemäß einem gleichschenkligen Dreieck, wodurch sich eine günstige Kräfteverteilung ergibt. Die Kammer belastet die bodenseitigen Abstandelemente 30, 30', 30" mit einer Druckbelastung, die deckenseitigen Abstandelemente werden durch Zugbelastung belastet, die Kammer ist hier an der oberen Seite der Gehäusefrontwand aufgehängt.

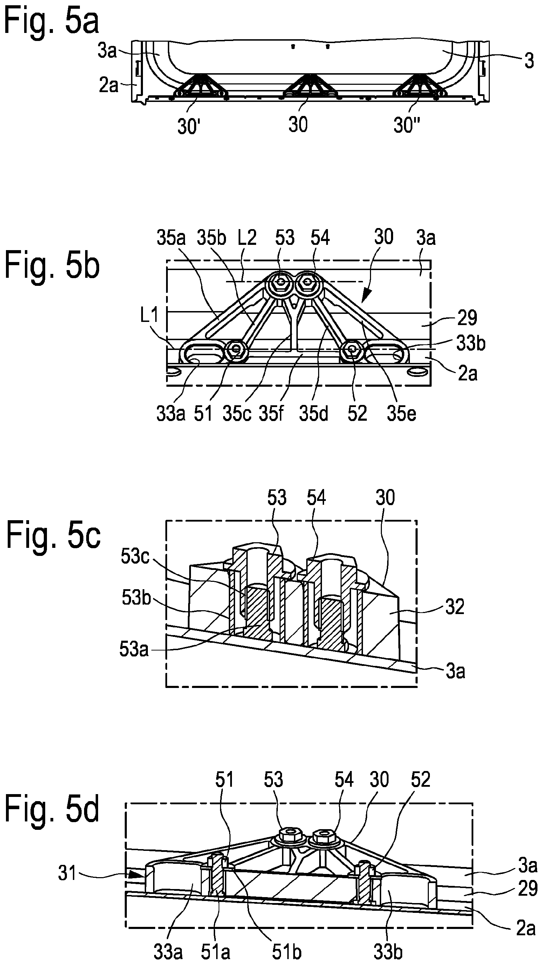

Der erste Verbindungsabschnitt 31 des bodenseitigen Abstandelements 30 ist hier ein balkenförmiger Bereich 31, siehe Fig. 5i, der die Gehäusefrontwand 2a im montierten Zustand kontaktiert und durch Befestigungsmittel 51, 52, 51', 52' gegen die Gehäusefrontwand 2a gelagert ist. Der erste Verbindungsabschnitt 31 weist zwei voneinander beabstandete und durch den stegförmigen Verbindungsabschnitt 35f verbundene Abschnitte mit den zylinderförmigen Löchern 34a und 34b auf, deren Zylinderachsen sich senkrecht zur Hauptebene des plattenförmigen Abstandelements 30 erstrecken. Die Löcher 34a und 34b werden zur Montage des zentralen Abstandelement 30 verwendet, wie in Fig. 5b bis 5d gezeigt ist.

Wie in Fig. 5b gezeigt ist, wird ein Abstandelement 30, 30', 30" durch ein plattenförmiges Bauteil mit etwa der Kontur eines gleichschenkligen Dreiecks gebildet. Die äußeren stegförmigen Abschnitte 35a, 35e schließen mit dem horizontal und am Boden des Dreiecks angeordneten stegförmigen Abschnitt 35f jeweils einen spitzen Winkel ein, insbesondere denselben spitzen Winkel, der hier ca. 41° beträgt. Im Treffpunkt der stegförmigen Abschnitte 35a, 35e mit dem stegförmigen Abschnitt 35f befinden sich, nebeneinander angrenzend, jeweils der oval-zylindrische Abschnitt mit Langloch 33a, 33b und der zylindrische Abschnitt mit zylinderförmigem Loch 34a, 34b. Die äußeren stegförmigen Abschnitte 35a, 35e starten jeweils vom oval-zylindrischen Abschnitt und münden in der Spitze des gleichschenkligen Dreiecks unter einem stumpfen Winkel, wobei die Spitze dort durch den ersten Verbindungsabschnitt 31 dargestellt wird, der aus zwei nebeneinanderliegenden zylindrischen Abschnitten mit jeweils einem zylinderförmigen Loch 37a, 37b gebildet wird. Zwei weitere stegförmige Abschnitte 35b, 35d schließen mit dem stegförmigen Abschnitt 35f einen Winkel von ca. 57° ein. Zentral im Abstandelement erstreckt dich der stegförmige Abschnitt 35c senkrecht zum stegförmigen Abschnitt 35f vertikal nach oben und mündet in der Spitze des Dreiecks, indem er sich y-förmig gabelt, und jede Teilgabel in einen der zylinderförmigen Abschnitte der Spitze mündet. Das Abstandelement 30 weist durch diese Strebenkonstruktion eine hohe mechanische Stabilität auf, insbesondere gegen die Druckbelastung von oben, ein geringes Gewicht, und einen hohen thermischen Widerstand gegen den Wärmestrom, der sich ausgehend vom ersten Verbindungsabschnitt 31 durch die Streben 35a, 35b, 35c, 35d, 35e zum zweiten Verbindungsabschnitt 32 ergibt. Die genannten Streben haben eine relativ große Länge und einen quer zum Wärmestrom gesehen geringen Querschnitt, was den Wärmestrom minimiert.

Wie in Fig. 5i gut erkennbar ist, weist das Abstandelement 30 an seiner Vorderseite eine Aussparung 36 auf, die zur Aufnahme eines anderen Bauteils ausgeformt ist, hier zur teilweisen Aufnahme der Dichtung 12, die im Spalt 29 angeordnet wird. Durch die Aussparung wird der im obigen Absatz benannte Querschnitt weiter reduziert und der thermische Widerstand erhöht. Die im Bereich der Aussparung 36 verlaufenden stegförmigen Streben 35a, 35b, 35c, 35d, 35e werden jeweils auch als ein dritter Abschnitt des Abstandelements aufgefasst, der jeweils einen ersten und zweiten Verbindungsabschnitt verbindet.

Fig. 5d zeigt eine perspektivische Querschnittsansicht des gehäuseseitigen ersten Verbindungsabschnitts 31 des zentral angeordneten Abstandelements der Fig. 5b, wobei der Querschnitt senkrecht zur Hauptebene des plattenförmigen Abstandelements und entlang der Linie L1 der Fig. 5b verläuft. Zwei ein Außengewinde tragende Metallstifte 51a sind mit der Rückseite der Gehäusefrontwand 2a so verschweißt, dass sie sich jeweils in ein Loch 34a, 34b des Abstandelements erstrecken. Eine Unterlegscheibe 51b und die auf den Metallstift 51a aufschraubbare Mutter 51 werden an jedem Loch 34a, 34b verwendet, um den ersten Verbindungsabschnitt 31 gegen die Gehäusefrontwand 2a zu pressen und so die Verbindung herzustellen.

Der zweite Verbindungsabschnitt 32 des bodenseitigen Abstandelements 30, 30', 30" wird durch einen schmaleren balkenförmigen Bereich 32 gebildet, siehe Fig. 5i, der die Kammerfrontwand 3a im montierten Zustand kontaktiert und durch Befestigungsmittel 53, 54 gegen die Kammerfrontwand 2a gelagert ist. Der zweite Verbindungsabschnitt 32 weist zwei zylinderförmige Löcher 37a, 37b auf, siehe Fig. 5h, deren Zylinderachsen sich senkrecht zur Hauptebene des plattenförmigen Abstandelements 30 erstrecken. Die Löcher 37a und 37b werden zur Montage der Abstandelemente 30, 30', 30" an der Kammerfrontwand 3a verwendet, wie in Fig. 5c gezeigt ist. In jedem der Löcher 37a und 37b ist eine passgenaue zylinderförmige Metallhülse 53b gelagert, deren Länge der Höhe des Abstandelements in dieser Richtung entspricht. Die mit der Kammerfrontwand 3a verschweißten Metallstifte 53a mit Außengewinde ragen in die Metallhülse hinein. Ein Mutternkopf 53, 54 wird mittels Gewinde 53c mit dem Metallstift 53a verschraubt und presst dabei gegen Metallhülse und Abstandelement 30, welches deshalb durch die Metallhülse entlastet wird.

Fig. 5e zeigt als einen Ausschnitt der Fig. 5a das dort links im Bild gezeigte Abstandelement 30'. Bei den dezentral, nämlich seitlich angeordneten bodenseitigen Abstandelementen 30' und 30" werden jeweils die am ersten Verbindungsabschnitt vorgesehenen Langlöcher verwendet, um das Abstandelement 30', 30" unter Tolerieren einer horizontalen Gleitbewegung an der Gehäusefrontwand zu befestigen. Auch die Abstandelemente 40, 40' an der Oberseite der Kammer weisen jeweils solch eine gleitende Befestigung mittels Langloch auf. Durch die Befestigung mittels der Langlöcher wird eine "schwimmende Befestigung" der Kammer am Gehäuse erzielt. Dieser Art der Lagerung ermöglicht es, thermisch bedingte Längenänderungen am Gehäuse auszugleichen bzw. die durch die thermisch bedingten Längenänderungen verursachten mechanischen Spannungen zu reduzieren. Lediglich das bodenseitig zentral angeordnete Abstandelement 30 ist nicht gleitend befestigt und garantiert die Positionierung der Kammer auch in Gegenwart thermisch bedingter Längenänderungen.

Fig. 5f zeigt eine perspektivische Querschnittsansicht des gehäuseseitigen ersten Verbindungsabschnitts des dezentral seitlich angeordneten Abstandelements 30' der Fig. 5e, wobei der Querschnitt senkrecht zur Hauptebene des plattenförmigen Abstandelements und entlang der Linie L3 der Fig. 5e verläuft. Zwei Metallstifte 51'a sind jeweils mit der Gehäusefrontwand 2a verschweißt und ragen durch das Langloch 33a, 33b hindurch. Eine zylinderförmige Gleithülse 51'b, 52'b umgibt den Metallstift 51'a, 52'a und endet in einem scheibenförmigen Hülsenkopf, der von der Mutter 51', 52' mit Druck beaufschlagt wird, die auf das Gewinde des Metallstifts 51'a, 52'a aufgeschraubt ist.

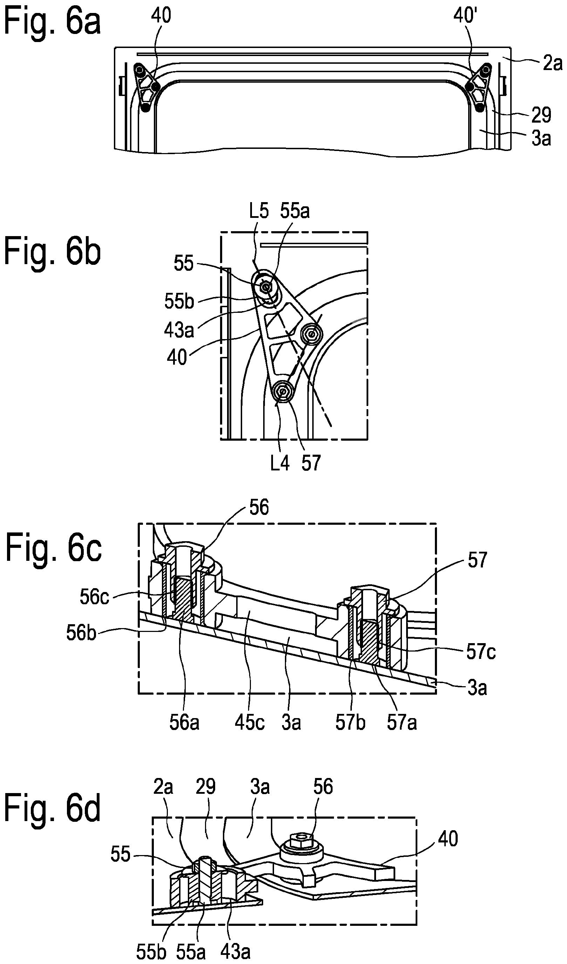

Fig. 6b zeigt als einen Ausschnitt der Fig. 6a das dort links im Bild sichtbare Abstandelement 40. Es weist einen ersten Verbindungsabschnitt 41 auf, der, wie in Fig. 6f und Fig. 6g erkennbar ist, als oval-zylindrischer Abschnitt 41 geformt ist, durch den sich das Langloch 43a erstreckt. Dieser Abschnitt 41 wird in der montierten Position gegen die Gehäusefrontwand 2a gelagert. Diese weist zu diesem Zweck einen mit ihr verschweißten Metallstift 55a auf, siehe Fig. 6d, den eine Gleithülse 55b umgibt, die in das Langloch 43a eingepasst ist, um dort mögliche mechanische Spannungen durch eine Gleitbewegung des Gehäuseabschnitts mit dem Metallstift 55a gegenüber dem mit der Kammerfrontwand 3a verbundenen Abstandelement 41 durchzuführen. Die Mutter 55 ist mit dem Metallstift 56a verschraubt und presst die Metallhülse so gegen die Gehäusefrontwand 2a, dass eine Gleitbewegung des Abstandelements 40 zwischen der Gehäusefrontwand 2a und der Metallhülse 55b ermöglicht wird.

Fig. 6c zeigt eine perspektivische Querschnittsansicht der kammerseitigen zweiten Verbindungsabschnitte 42, 43 des Abstandelements 40 der Fig. 6b, wobei der Querschnitt senkrecht zur Hauptebene des plattenförmigen Abstandelements und entlang der Linie L4 der Fig. 6b verläuft. Die als "zweite Verbindungsabschnitte" bezeichneten Abschnitte 42, 43 verbinden das Abstandelement 40 mit der Kammerfrontwand 3a. Die Verbindungsabschnitte 42 und 43 sind zylindrische Abschnitte des Abstandelements 40, wie in Fig. 6f, 6g zu sehen, durch die sich jeweils ein zylinderförmiges Loch 44a, 44b erstreckt. Diese dienen der Befestigung des Abstandelements 40 an der Kammerfrontwand 3a. Diese weist zu diesem Zweck zwei mit ihr verschweißte Metallstifte 56a, 57a auf. Jeder dieser Stifte erstreckt sich in ein Loch 44a, 44b des Abstandselements 40. In jedes Loch ist eine Metallhülse 56b, 57b eingepasst, die das Loch jeweils einfasst. Mittels einer Mutter 56, 57, die sich jeweils in das Loch erstreckt, die auf der Metallhülse aufliegt und die mit dem Außengewinde 56c, 57c des Metallstifts 56a, 57a verbunden ist, ist jeder der Verbindungsabschnitte 42, 43 zwischen Metallhülse 56b, 57b und der Kammerfrontwand 3a gelagert und so befestigt, dass die Druckkraft von der Metallhülse aufgenommen wird und das Abstandselement entlastet wird.

Fig. 6e zeigt eine Aufsicht auf das Abstandselement der Fig. 6b, in der die stegförmigen Abschnitte 45a, 45b, 45c, 45d zwischen den Verbindungsabschnitten 41, 42, 43 bezeichnet sind. Der Steg 45a verbindet den Verbindungsabschnitt 41 mit dem Verbindungsabschnitt 43, der Steg 45b verbindet den Verbindungsabschnitt 41 mit dem Verbindungsabschnitt 42, der Steg 45c verbindet den Verbindungsabschnitt 42 mit dem Verbindungsabschnitt 43, Steg 45d verbindet den Verbindungsabschnitt 42 mit der Strebe 45a. Auf diese Weise ist eine leichtgewichtige, mechanisch belastbare Konstruktion geschaffen, deren thermischer Widerstand wegen des geringen Querschnitts der Stege 45a, 45b senkrecht zur Wärmestromrichtung zwischen dem ersten und zweiten Verbindungsabschnitt und der relativ großen Länge dieser Stege maximiert ist. Wie man in Fig. 6c gut erkennen kann, ist der Steg 45c -anders als der Steg 35f des Abstandselements 30 (Fig. 5f) nicht in Kontakt mit dem entsprechenden Frontwandblech: in Fig. 6c ist der Steg durch einen Spalt von der Kammerfrontwand 3a getrennt. Die Abschnitte 42 und 43 des Abstandselements werden jeder für sich an der Kammerfrontwand gelagert und deshalb, anders als der Balkenförmige Bereich 31 in Fig. 5f, als zwei unterschiedliche Verbindungsabschnitte angesehen.

Das in der anderen oberen Ecke der Kammerfrontwand 3a angeordnete Abstandselement 40' ist analog dem Abstandselement 40 ausgebildet, aber spiegelbildlich zu diesem, und in derselben Weise mit Kammerfrontwand 3a und der Gehäusefrontwand 2a verbunden.

Fig. 7 zeigt ausschnittsweise eine seitliche Querschnittsansicht des in Fig. 1a gezeigten Inkubators, wobei der Querschnitt parallel zu einer Seitenwand des Inkubators verläuft und einen oberen Frontbereich des Inkubators zeigt. Dort ist ein oberer Bereich der Gehäusetüre 4 ausschnittsweise gezeigt, in der geschlossenen Position der Außentüre 4, in der dieser obere Bereich der Außentüre mittels Dichtungen an der Gehäusefrontwand 2a anliegt. Die Kammer 3 weist eine Kammertüre 10 auf, die hier auch geschlossen ist. Die Gehäusefrontwand 2a und die Kammerfrontwand 3a sind durch die erste Dichtung 12 voneinander getrennt, die um die Kammeröffnung umläuft. Die erste Dichtung ist aus Silikonschaum gefertigt. Die Außentüre 4 weist eine Innenseite 4a mit einer zweiten Dichtung 14 auf, die im geschlossenen Zustand der Außentüre an der ersten Dichtung 12 anliegt und um die Kammeröffnung 3z sowie die Gehäuseöffnung 2z umläuft.

Eine solche erfindungsgemäße Labortemperiervorrichtung bietet den Vorteil, dass der bei geschlossener Außentüre zwischen Kammertüre und Gehäusetüre gebildete Zwischenraum 60 nicht mit dem Gehäuse in Kontakt steht. Dies ist darin begründet, dass die zweite Dichtung im geschlossenen Zustand der Außentüre durch das lückenlose Kontaktieren ersten Dichtung den Zwischenraum seitlich begrenzt. Jeglicher Kontakt des im Zwischenraum 60 enthaltenen, von der Kammer erwärmten Luftgemisches mit der nicht-temperierten Außenwand des Gehäuses und somit jeglicher konvektionsbedingte Wärmetransport zwischen dem Zwischenraum 60 und dem Gehäuse ist somit unmöglich.

Die erste Dichtung schließt den Spalt 29 zwischen Gehäusefrontwand 2a und der Kammerfrontwand 3a. Die Spaltbreite d beträgt hier d1=14 mm. Die Breite der ersten Dichtung 12 in Richtung d1 beträgt etwas mehr als 14 mm, da die planaren Blechenden der Gehäusefrontwand 2a und der Kammerfrontwand 3a jeweils von gegenüberliegenden Seiten in eine entsprechende Nut der ersten Dichtung eingreifen, wodurch sich eine Formschlussverbindung zwischen erster Dichtung und der Gehäusefrontwand 2a und der Kammerfrontwand 3a ergibt. Die minimale Dicke der ersten Dichtung liegt hier bei d2 = 3,0 mm. Durch die genannten bevorzugten Ausgestaltungen (maximale Wärmestromstrecke in Richtung der Spaltbreite und minimaler wärmeleitender Querschnittsenkrecht zu dieser Richtung) ist der thermische Widerstand der ersten Dichtung 12 maximal.

Die zweite Dichtung weist einen näher an der Außentüre gelegenen, umlaufenden ersten Dichtungsbereich 14a auf, der ein höheres Elastizitätsmodul aufweist als der zweite, integral mit dem ersten Dichtungsbereich verbundene zweite Dichtungsbereich 14b, der insbesondere weicher ist und der in der geschlossenen Position der Außentüre näher an der ersten Dichtung liegt und diese kontaktiert. Durch den weicheren Dichtungsbereich 14b zwischen Außentüre 4 und Gehäuse 2 lässt sich der Zwischenraum zwischen Außentüre und Kammertüre besser abdichten, zudem werden die zum Schließen der Kammertüre erforderlichen Kräfte reduziert, so dass die Bedienung für den Benutzer leichter wird. Der erste Dichtungsbereich ist unter Verwendung von Silikon gefertigt, der zweite Dichtungsbereich 14b ist unter Verwendung eines Silikonschaums gefertigt.

Durch den abgeschlossenen Zwischenraum zwischen Außentüre 4 und Kammertüre 10, der nicht mit der relativ kühlen Gehäusefrontwand in Kontakt steht, wird der Wärmestrom durch den Frontbereich des Inkubators reduziert und die thermische Entkopplung zwischen Kammer und Außenwelt wird weiter verbessert.

Die Außentüre weist eine beheizte Innenwand 4a auf. Diese wird, ohne die äußere Wand 4b der Außentüre direkt zu berühren, durch mehrere Abstandselemente 4c an der äußeren Wand 4b der Außentüre gehalten. Ein Abstandselement 4c ist vorzugsweise aus einem Material mit einer Wärmeleitfähigkeit kleiner als 15 W(mK) gebildet, insbesondere aus einem Hochleistungskunststoff, insbesondere PPS. Dadurch wird die thermische Entkopplung der Kammer von der Umgebung weiter verbessert.

Der um die Kammerfrontwand 2a umlaufende Wand-Endabschnitt 2a' der Gehäusefrontwand bildet die Gehäuseöffnung 2z, und die Kammerfrontwand 3a bildet einen Flansch um die Kammeröffnung 3z, der in einem um die Kammeröffnung umlaufenden Wand-Endabschnitt 3a' endet. Der Wand-Endabschnitt 2a' der Gehäusefrontwand und der Wand-Endabschnitt 3a' der Kammerfrontwand weisen jeweils eine Dicke von ca. 2,0 mm auf und liegen einander gegenüber, um den Spalt 29 zu bilden. Durch die erste Dichtung 12 sind die ohnehin bereits dünnen Wand-Endabschnitte miteinander verbunden. Der Wand-Endabschnitt 2a' der Gehäusefrontwand und der Wand-Endabschnitt 3a' der Kammerfrontwand liegen hier zudem in derselben Ebene. Der Wärmeeintrag des Wand-Endabschnitts 3a' der Kammerfrontwand in die Dichtung ist wegen der geringen Wanddicke gering, so dass die Wärmeübertragung weiter reduziert wird. Durch diese Gestaltung ist insbesondere auch die Wärmeübertragung zwischen Wand-Endabschnitt 2a' der Gehäusefrontwand und der Wand-Endabschnitt 3a' der Kammerfrontwand mittels Wärmestrahlung minimiert und die thermische Entkopplung zwischen Kammer und Gehäuse weiter verbessert.

Durch die hier beschriebenen Maßnahmen der thermischen Entkopplung von Kammer und Gehäuse konnte überraschender Weise der Energieverbrauch des erfindungsgemäßen Inkubators gegenüber einem Inkubator der aktuellen Generation um etwa 50% gesenkt werden, was die Effizienz der genannten Maßnahmen verdeutlicht.

**Bezugszeichenliste**

1: Labortemperierschrank, Inkubator
2: Gehäuse, Gehäusewand
2a: Frontwand des Gehäuses
2a': Wand-Endabschnitt der Gehäusefrontwand
2b: Rückwand des Gehäuses
2c: Seitenwände des Gehäuses
2d: obere Außenseite des Gehäuses
2e: Gehäusebodenwand
2z: Gehäuseöffnung
3: Kammer / Kammerinnenraum
3a: Kammerfrontwand
3a': Wand-Endabschnitt der Kammerfrontwand
3b: Kammerrückwand
3c: Seitenwand der Kammer
3d: Deckenwand der Kammer
3e: Kammerbodenwand
3z: Kammeröffnung
4: Außenseite der Gehäusetür/ Außentür
4a: beheizte Innenseite der Gehäusetür / Außentür
4b: äußere Wand der Gehäusetür/ Außentür

4c: Abstandselement
5: Benutzerschnittstelleneinrichtung
6: Griff Außentür
7: Verriegelungseinrichtung
7a: gehäuseseitiger Halteabschnitt der Verriegelungseinrichtung
7b: gehäusetürseitiger Halteabschnitt der Verriegelungseinrichtung
8: Sockel
9: Scharnier
10: Kammertür; transparent
11: elastische Dichtung
12: elastische Dichtung; erste Dichtung
13: magnetischer Handverschluss
14: elastische Dichtung; zweite Dichtung
14a: erster Dichtungsbereich aus ungeschäumten Silikon
14b: weicherer Dichtungsbereich aus Silikonschaum; zweiter Dichtungsbereich
15: Scharnier
16: doppelte Außenrückwand
17: Griff der Rückwand
18: Bauteile zur Temperaturregelung
19: Isoliereinrichtung
19a: Nadelvliesstreifen der Isoliereinrichtung; umläuft Kammer
19b: U-förmig gebogenes Isolierelement der Isoliereinrichtung 19 aus Glaswolle
19c: Isolierplatte der Isoliereinrichtung 19 aus PIR Schaum
20: Zugangsport
20': Zugangsport; Abstandselement
20h: Öffnung des Zugangsports in der Kammerrückwand
20'h: Öffnung des Zugangsports in der Kammerrückwand
20a: Gewindering
20b: Gewindering
20c: Gewindering
21: äußeres Ende des Zugangsports; erster Verbindungsabschnitt
22: Flansch des Zugangsports; zweiter Verbindungsabschnitt
25: Stopfen des Zugangsports
29: Spalt
30: Abstandselement
30': Abstandselement
30": Abstandselement
31: erster Verbindungsabschnitt
32: zweiter Verbindungsabschnitt
33a: Langloch
33b: Langloch
34a: zylinderförmige Löcher
34b: zylinderförmige Löcher
35a: stegförmiger Abschnitt
35b: stegförmiger Abschnitt
35c: stegförmiger Abschnitt
35d: stegförmiger Abschnitt

35e: stegförmiger Abschnitt

35f: stegförmiger Abschnitt

36: Aussparung

37a: zylinderförmiges Loch

37b: zylinderförmiges Loch

40: Abstandselement

40': Abstandselement

41: erster Verbindungsabschnitt

42: zweiter Verbindungsabschnitt

43: dritter Verbindungsabschnitt

43a: Langloch

44a: zylinderförmiges Loch

44b: zylinderförmiges Loch

45a: stegförmiger Abschnitt; Strebe; Stege

45b: stegförmiger Abschnitt

45c: stegförmiger Abschnitt

45d: stegförmiger Abschnitt

51: Befestigungsmittel; Mutter

51': Befestigungsmittel; Mutter

51a: Metallstift

51a': Metallstift

51b: Unterlegscheibe

51'b: zylinderförmige Gleithülse

52: Befestigungsmittel

52': Befestigungsmittel; Mutter

52'a: Metallstift

52'b: zylinderförmige Gleithülse

53: Befestigungsmittel; Mutternkopf

53a: Metallstift

53b: Metallhülse

53c: Gewinde

54: Mutternkopf

55: Mutter

55a: Metallstift

55b: Gleithülse; Metallhülse

56: Mutter

56a: Metallstift

56b: Metallhülse

56c: Gewinde

57: Mutter

57a: Metallstift

57b: Metallhülse

57c: Gewinde

60: Zwischenraum zwischen Kammertüre und Gehäusetüre

**Patentansprüche**

1.  Labortemperiervorrichtung (1), insbesondere Inkubator für Zellkulturen, zum temperierten Lagern von Laborproben, aufweisend
    ein Gehäuse (2) mit einem von mindestens einer Gehäusewand umgebenen Gehäuseinneren,
    eine im Gehäuse angeordnete temperierbare Kammer (3) mit einem von mindestens einer Kammerwand umgebenen Kammerinneren zur Aufnahme der Laborproben,

mehrere Abstandselemente (30; 30'; 30"; 40; 40'; 20; 20'), wobei ein Abstandselement mindestens einen ersten Verbindungsabschnitt (31; 41; 21) aufweist, mittels dem das Abstandselement und das Gehäuse verbunden sind und mindestens einen vom ersten Verbindungsabschnitt beabstandeten zweiten Verbindungsabschnitt (32; 42; 22) aufweist, mittels dem das Abstandselement und die Kammer verbunden sind, so dass die Kammer mittels der Abstandselemente beabstandet zum Gehäuse gehalten wird,
wobei die Abstandselemente (30; 30'; 30"; 40; 40'; 20; 20') jeweils unter Verwendung eines Materials mit einer Wärmeleitfähigkeit kleiner als 15 W/(mK) gebildet sind.

2.  Labortemperiervorrichtung gemäß Anspruch 1, wobei mindestens eines der Abstandselemente unter Verwendung eines Hochleistungskunststoffs gefertigt ist, der Betriebstemperaturen von 180 °C toleriert.

3.  Labortemperiervorrichtung gemäß Anspruch 2, wobei der Hochleistungskunststoff Polyphenylensulfid (PPS) oder Polyetheretherketon (PEEK) ist.

4.  Labortemperiervorrichtung gemäß Anspruch 2 oder 3, wobei der Hochleistungskunststoff zur mechanischen Verstärkung Faserzusätze aufweist, insbesondere Glasfaser.

5.  Labortemperiervorrichtung gemäß einem der vorangehenden Ansprüche, wobei mindestens ein Abstandselement ein plattenförmiges Bauteil ist.

6.  Labortemperiervorrichtung gemäß einem der vorangehenden Ansprüche, wobei mindestens ein Abstandselement mindestens eine Aussparung und/oder mindestens einen Hohlraum aufweist.

7.  Labortemperiervorrichtung gemäß einem der vorangehenden Ansprüche, wobei mindestens ein Abstandselement mehrere stegförmige Abschnitte aufweist.

8.  Labortemperiervorrichtung gemäß Anspruch 7, wobei mindestens ein stegförmiger Abschnitt den ersten Verbindungsabschnitt und den zweiten Verbindungsabschnitt verbindet und/oder wobei mindestens ein stegförmiger Abschnitt zwei erste Verbindungsabschnitte oder zwei zweite Verbindungsabschnitte verbindet.

9.  Labortemperiervorrichtung gemäß einem der vorangehenden Ansprüche, wobei
    das Gehäuse eine Gehäusefrontwand (2a) aufweist und die Kammer eine Kammerfrontwand (3a) aufweist, wobei Gehäusefrontwand und die Kammerfrontwand durch eine erste Dichtung (12) voneinander getrennt sind, die um die Kammeröffnung (3z) umläuft,

wobei eine Außentüre des Gehäuses eine Innenseite mit einer zweiten Dichtung (14) aufweist, die im geschlossenen Zustand der Außentüre an der ersten Dichtung anliegt und um die Kammeröffnung umläuft.

10. Labortemperiervorrichtung, insbesondere Inkubator für Zellkulturen, aufweisend

ein Gehäuse mit einer Außentüre und einer durch die Außentüre verschließbaren Gehäuseöffnung, die in einer Gehäusefrontwand ausgebildet ist,

eine im Gehäuse angeordnete, ein Kammerinneres umgebende Kammer (3) mit einer Kammeröffnung (3z), die in einer Kammerfrontwand (3a) ausgebildet ist,

wobei die Gehäusefrontwand (2a) und die Kammerfrontwand (3a) durch eine erste Dichtung (12) voneinander getrennt sind, die um die Kammeröffnung umläuft,

wobei die Außentüre (4) eine Innenseite (4) mit einer zweiten Dichtung (14) aufweist, die im geschlossenen Zustand der Außentüre an der ersten Dichtung (12) anliegt und um die Kammeröffnung (3z) umläuft.

11. Labortemperiervorrichtung gemäß Anspruch 10, wobei die erste Dichtung eine minimale Materialdicke (d2) aufweist, die, gemessen senkrecht zur Gehäusefrontwand, geringer ist als die Spaltbreite (d1) des Spalts zwischen der Gehäusefrontwand und der Kammerfrontwand.

12. Labortemperiervorrichtung gemäß Anspruch 10 oder 11, wobei die zweite Dichtung in der geschlossenen Position der Außentüre auch an der Gehäusefrontwand (2a) anliegt.

13. Labortemperiervorrichtung gemäß Anspruch 10, 11 oder 12, wobei die zweite Dichtung (14) eine beheizte Innenseite (4a) der Außentüre (4) von der Außenwand der Außentüre trennt.

14. Labortemperiervorrichtung gemäß Anspruch 10, 11, 12 oder 13, wobei diese mehrere Abstandselemente aufweist, wobei ein Abstandselement mindestens einen ersten Verbindungsabschnitt aufweist, mittels dem das Abstandselement und das Gehäuse verbunden sind und mindestens einen vom ersten Verbindungsabschnitt beabstandeten zweiten Verbindungsabschnitt aufweist, mittels dem das Abstandselement und die Kammer verbunden sind, so dass die Kammer mittels der Abstandselemente beabstandet zum Gehäuse gehalten wird, wobei die Abstandselemente jeweils unter Verwendung eines Materials mit einer Wärmeleitfähigkeit kleiner als 15 W/(mK) gebildet sind.

15. Labortemperiervorrichtung gemäß einem der vorangehenden Ansprüche 1 bis 14, die ein Inkubator (1), insbesondere ein $CO_2$-Inkubator für Zellkulturen ist.

Fig. 1a

Fig. 1b

Fig. 1c

# Fig. 2a

# Fig. 2b

# Fig. 3a

# Fig. 3b

Fig. 4a

Fig. 4b

# Fig. 5a

# Fig. 5b

# Fig. 5c

# Fig. 5d

Fig. 5e

53    54    30'
3a
29
2a
51'a
L3

51'  51'b  34a    34b  52'

Fig. 5f

51'    53    54  30'
52'
3a
29
2a

51'b    51'a  33a  34a    34b  33b

Fig. 5g

53    54
3a
29
2a
52'b

33a  51'a  51'b  34a  34b  33b  52'a

Fig. 5h

37a  37b
36  30, 30', 30"

Fig. 5i

32
36
31
30, 30', 30"

**Fig. 6a**

**Fig. 6b**

**Fig. 6c**

**Fig. 6d**

Fig. 6e

Fig. 6f

Fig. 6g

Fig. 7

EP 3 552 703 A1

Europäisches Patentamt
European Patent Office
Office européen des brevets

# EUROPÄISCHER RECHERCHENBERICHT

**Nummer der Anmeldung**

EP 18 16 6337

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 22 59 377 A1 (HERAEUS GMBH W C) 27. Juni 1974 (1974-06-27) | 1 | INV. B01L1/00 C12M1/00 |
| Y | * Seite 3, Absatz 4 * * Seite 5, Absatz 4; Abbildung 6 * | 2,3 | |
| Y | DE 10 2014 212356 B3 (MEMMERT GMBH & CO KG [DE]) 27. August 2015 (2015-08-27) * Absatz [0015]; Abbildungen 3a,3b * | 2,3 | |
| X | US 2 233 394 A (ASHBAUGH JOHN H) 4. März 1941 (1941-03-04) | 10-12 | |
| A | * Abbildung 2 * | 13-15 | |
| A | EP 0 154 536 A2 (MALLINCKRODT INC [US]) 11. September 1985 (1985-09-11) * Seite 11 - Seite 12; Abbildung 4 * | 1 | |
| A | FR 705 807 A (M. RUDOLF HORCH) 12. Juni 1931 (1931-06-12) * das ganze Dokument * | 1-15 | |

**RECHERCHIERTE SACHGEBIETE (IPC)**

B01L
C12M

Der vorliegende Recherchenbericht wurde für alle Patentansprüche erstellt

| Recherchenort | Abschlußdatum der Recherche | Prüfer |
|---|---|---|
| Berlin | 20. September 2018 | Busuiocescu, Bogdan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&.................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 16 6337

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-09-2018

| Im Recherchenbericht angeführtes Patentdokument | Datum der Veröffentlichung | Mitglied(er) der Patentfamilie | Datum der Veröffentlichung |
|---|---|---|---|
| DE 2259377 A1 | 27-06-1974 | KEINE | |
| DE 102014212356 B3 | 27-08-2015 | DE 102014212356 B3<br>EP 3161122 A1<br>WO 2015197511 A1 | 27-08-2015<br>03-05-2017<br>30-12-2015 |
| US 2233394 A | 04-03-1941 | KEINE | |
| EP 0154536 A2 | 11-09-1985 | KEINE | |
| FR 705807 A | 12-06-1931 | KEINE | |

EPO FORM P0461

Für nähere Einzelheiten zu diesem Anhang : siehe Amtsblatt des Europäischen Patentamts, Nr.12/82